# EUROPEAN PATENT SPECIFICATION

(11) **EP 3 833 431 B1**
(45) Date of publication and mention of the grant of the patent: **27.11.2024**
(21) Application number: 19847365.4
(22) Date of filing: 09.08.2019
(51) Int. Cl.: A61N 5/06, A61B 18/18, A61B 18/20, A61B 90/00

(54) **DELIVERY OF ENERGY TO A TARGET REGION OF A PATIENT'S BODY TO SATISFY THERAPEUTIC REQUIREMENTS PRECISELY**
ABGABE VON ENERGIE AN EINEN ZIELBEREICH EINES PATIENTENKÖRPERS ZUR PRÄZISEN ERFÜLLUNG THERAPEUTISCHER ANFORDERUNGEN
FOURNITURE D'ÉNERGIE À UNE RÉGION CIBLE DU CORPS D'UN PATIENT POUR SATISFAIRE PRÉCISÉMENT À DES EXIGENCES THÉRAPEUTIQUES

(30) Priority: 09.08.2018 US 201862716449 P
(43) Date of publication of application: 16.06.2021
(73) Proprietor: The General Hospital Corporation, Boston, Massachusetts 02114 (US)
(72) Inventor: LEVIN, Philip, Storrs, Conneticut 06268 (US)
(74) Representative: Leach, Sean Adam
(86) International application number: PCT/US2019/045945
(87) International publication number: WO 2020/033855

(56) References cited:
- WO-A1-2016/201557
- KR-A- 20180 085 250
- US-A1- 2006 217 688
- US-A1- 2008 142 739
- US-A1- 2010 094 265
- US-A1- 2010 094 265
- US-A1- 2012 150 264
- US-A1- 2013 096 546
- US-A1- 2013 231 720
- US-A1- 2014 005 756
- US-A1- 2016 074 222
- US-A1- 2017 004 267
- US-A1- 2017 281 981
- US-A1- 2018 325 622

## Description

### Technical Field

The present disclosure relates generally to delivery of therapeutic energy and, more particularly, to systems and methods that deliver energy to a target region of a patient's body (of arbitrary shape and/or size) to precisely satisfy therapeutic requirements.

### Background

Types of energy, like optical energy, are increasingly used to treat a wide variety of medical conditions. For example, the U.S. Food and Drug Administration (FDA) has approved the use of optical energy to treat cancer and other similar conditions, relieve pain due to musculoskeletal disorders and autoimmune disorders, perform cosmetic procedures, and the like. However, optical energy has not been widely employed for certain applications requiring energy delivery because the delivery of the optical energy is time consuming, expensive, and not particularly precise. KR20180085250A relates to a device and a method for ultraviolet target ray treatment of a light-sensitive skin disease using 3D scanning technology and feedback. The device and method are capable of removing a bad effect applied to the normal skin of a lesion boundary surface with the control of a light irradiation amount. The treatment efficiency is improved by accurately recognizing a curvature of a lesion part with 3D scanning and detecting the lesion treatment light for feedback. The device for ultraviolet target ray treatment of a light-sensitive skin disease uses 3D scanning technology and feedback comprising a 3D scanner, an ultraviolet camera, and a composite controller. The 3D scanner transmits a pattern image for a lesion of a subject by photographing the subject after projecting scan light to the lesion of the subject, and irradiates the treatment light to a target lesion depending on a predetermined treatment light condition. The ultraviolet camera outputs an ultraviolet image of the lesion part by photographing ultraviolet rays to the lesion part. The composite controller generates a 3D lesion image including the lesion after receiving the pattern image, sets the treatment light condition by using a 3D shape of the target lesion included in the 3D lesion image, measures distribution of a light irradiation amount in the lesion part by receiving the ultraviolet image of the lesion part, and changes and outputs the treatment light condition to uniform the light irradiation amount.

US2013/231720 A1 relates to a method for irradiating or treating areas of the body with electromagnetic radiation from a radiation source, the area of the body including at least one irregularly outlined treatment area, which is determined and irradiated. The invention is characterized in that the area of the body is divided up into a number of subareas, which at least partly contain the treatment area, and a proportion of the treatment area contained in each sub-area is irradiated with a radiation dose sequentially or in a scrolled manner or step-by-step or in a targeted manner. Irradiation power can be modulated at high frequency with help of micromirrors.

US2012/150264 A1 relates to a skin radiation apparatus comprising a photon radiation unit for generating a line-shaped radiation pattern extending in a first direction. The photon radiation unit comprising a photon radiation source, a movement facility for moving the line shaped radiation pattern in a second direction transverse to the first direction, detection unit for detecting a skin condition profile, a control unit for controlling an power density distribution for the line-shaped radiation pattern dependent on the skin condition profile detected by the detection unit.

### Summary

The presently claimed invention provides a system as set out in claim 1. According to the present disclosure, an energy beam is manipulated and scanned to fill a treatment region of any size or shape to precisely, specifically, and rapidly deliver a required dose and/or fluence.

### Brief Description of the Drawings

The foregoing and other features of the present disclosure will become apparent to those skilled in the art to which the present disclosure relates upon reading the following description with reference to the accompanying drawings, in which:
FIG. 1 is a diagram illustrating a system that delivers a dose profile and/or a fluence profile to a target region of a patient's body of a shape and/or a size according to an aspect of the present disclosure;
FIG. 2 includes two diagrams illustrating examples of beam projection along a path that can be employed by the system of FIG. 1;
FIG. 3 is a process flow diagram illustrating a method for delivering a dose profile and/or a fluence profile to a target region of a patient's body of a shape and/or a size according to another aspect of the present disclosure;
FIG. 4 shows a normalized Gaussian beam profile;
FIG. 5 shows a summation of two neighboring Gaussian beams;
FIG. 6 shows the translation of a Gaussian beam from left to right and the associated accumulated dose that is delivered to a target region;
FIG. 7 shows the relationship between the beam spot size and the sharpness of the accumulated dose profile;
FIG. 8 shows the relationship between the separation of adjacent scan paths and the uniformity of the accumulated dose;
FIG. 9 shows different degrees of uniformity used for different therapeutic applications;
FIGS. 10 and 11 show the attenuation of energy as a function of depth;
FIGS. 12 and 13 show a triangular treatment zone achieved by translation of a Gaussian beam;
FIGS. 14, 15, 16, and 17 show the application of treatment to a patient's thyroid; and
FIGS. 18 and 19 show fiducial markers being used to effect precise registration of the beam path with the intended surface treatment zone or the intended deep tissue treatment zone.

### Detailed Description

### Definitions

Unless defined otherwise, all technical and scientific terms used herein have the same meaning as is commonly understood by one of skill in the art to which the present disclosure pertains.

In the context of the present disclosure, the singular forms "a," "an" and "the" can also include the plural forms, unless the context clearly indicates otherwise.

The terms "comprises" and/or "comprising," as used herein, can specify the presence of stated features, steps, operations, elements, and/or components, but do not preclude the presence or addition of one or more other features, steps, operations, elements, components, and/or groups.

As used herein, the term "and/or" can include any and all combinations of one or more of the associated listed items.

Additionally, although the terms "first," "second," etc. may be used herein to describe various elements, these elements should not be limited by these terms. These terms are only used to distinguish one element from another. Thus, a "first" element discussed below could also be termed a "second" element without departing from the teachings of the present disclosure.

As used herein, the term "energy" can be used to represent radiant energy that travels by waves or particles, particularly electromagnetic radiation (visible and/or invisible). Energy that is used in connection with light (a type of electromagnetic radiation) can be referred to as optical energy. "Energy beams," "electromagnetic beams," "optical beams," and the like as used herein can refer to radiant energy that travels by waves or particles and that remains essentially concentrated around a mean axis upon free propagation or that is guided by suitable structures such as lenses or reflectors.

As used herein, the term "energy source" can refer to something that can be configured to provide/generate an energy beam. In the case of optical energy, the energy source can include, for example, at least one laser and/or light source and can be configured to permit spectral adjustment and/or spectral modulation of the energy beam.

As used herein, the term "energy beam" can refer to energy being sent in a direction. The energy beam can be pulsed and/or continuous. The energy beam can be projected with a shape (circle, rectangle, square, hexagon, triangle, etc.) and/or a size. Additionally, the energy beam can be collimated, diverging, converging, or have a focal length. The energy beam can be of any beam profile type. In some instances, the energy beam can possess a Gaussian intensity profile or the energy beam can possess a non-Gaussian intensity profile.

As used herein, the term "dose" can refer to the total energy delivered to a target region. Delivery of energy to a patient can have certain dose requirements. In some instances, the term dose can include dosage (dose per unit area).

As used herein, the term "dose profile" can refer to the 2D or 3D pattern of accumulated dose within a target region, after treatment is completed. The "dose profile" can also refer to the pattern of dose occurring along or to the sides of a specified path of a beam, and to a specified depth, as the beam scans within the target region.

As used herein, the term "fluence" can refer to the energy that passes through a surface area of a target region. Delivery of energy to a patient can have certain fluence requirements.

As used herein, the term "fluence profile" can refer to the 2D or 3D pattern of accumulated fluence within a target region, after treatment is completed. The "fluence profile" can also refer to the pattern of fluence occurring along or to the sides of a specified path of a beam, and to a specified depth, as the beam scans within the target region.

As used herein, the term "delivery characteristic" can refer to one or more properties of the energy beam. The delivery characteristic can be based on the dose profile and/or the fluence profile and can be variable along the path. For example, the delivery characteristic can include a beam scan rate, a beam path repetition number, a number of repetitions of an area of the path, a beam power, a beam power modulation pattern and/or rate, a beam size, a beam collimation, divergence, convergence, or focus, a beam intensity profile, a beam shape, or the like.

As used herein, the term "target region" can refer to a portion of a patient's body of an arbitrary shape and/or size to be treated with energy. The target region can include an area, a surface area, a volume, or the like. In certain examples, the target region can be large and/or complex.

As used herein, the term "path" can refer to a pattern in space along which the energy beam is translated. The path may be continuous or discontinuous. A discontinuous path comprises two or more sub-paths that are separated in space from each other. In some instances, the path can include one or more guides, in, on, near, or surrounding the patient, for the energy beam. In some instances, the path can include a marking in, on, or below the patient's skin (e.g., a drawing, an outline, a fiducial marker, or the like).

As used herein, the term "duration" can refer to a time the target region is exposed to the energy. In some instances, different portions of the target region can be exposed to the energy for different times.

As used herein, the terms "subject" and "patient" can be used interchangeably and refer to any warm-blooded organism including, but not limited to, a human being, a pig, a rat, a mouse, a dog, a cat, a goat, a sheep, a horse, a monkey, an ape, a rabbit, a cow, *etc.*

### Overview

The present disclosure describes the delivery of energy to a treatment region of a patient's body (a line, a curve, an area, surface area, and/or volume of arbitrary shape or size) to precisely satisfy therapeutic dose requirements and/or fluence requirements. A clinical outcome is usually sensitive to both dose and fluence (such that fluence levels that are too high can be acutely dangerous, while fluence levels that are too low might reside below a threshold for effectiveness, if such a threshold exists). The present disclosure empowers clinicians and other medical professionals to precisely achieve a required total dose and/or a required average, minimum, or maximum fluence over the entire treatment region (however large or irregularly shaped that region may be). Advantageously, the energy can be delivered to the area or volume of the patient's body without requiring a large and expensive energy source. Systems and methods described herein employ a beam, of smaller dimensions than the treatment region itself, to scan over a path through the treatment region. Other factors that impact efficacy and safety, such as the following, may also be precisely controlled within the treatment region: the dose profile and/or fluence profile; certain important dynamic parameters, such as the scan rate, path repetition rate, power modulation rate and the temporal modulation shape (cw, step function, saw-tooth curve, etc.); spectral content of the beam; and treatment depth. In other words, an energy profile in time, space, and wavelength may be precisely delivered.

### Systems

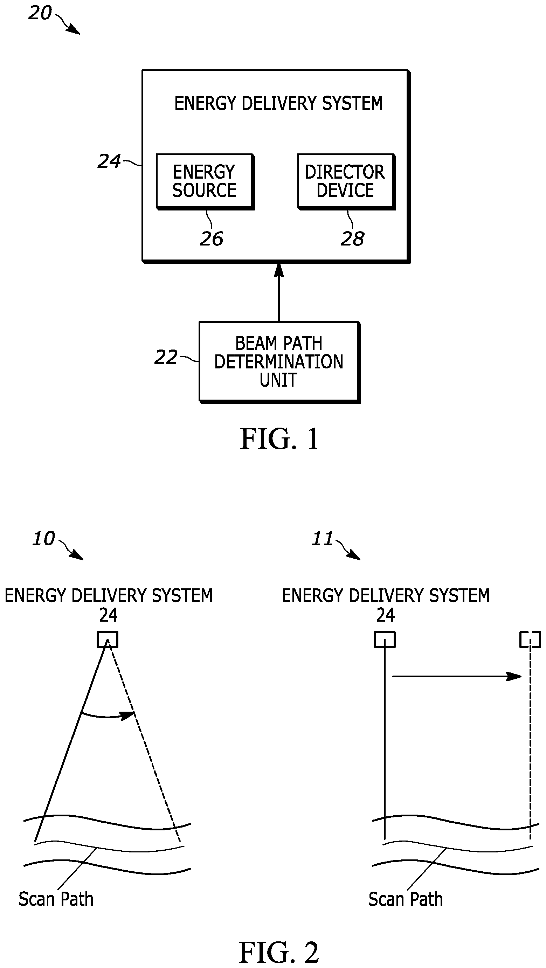

FIG. 1 shows a system 20 that delivers a dose profile and/or a fluence profile (corresponding to the delivery of energy) to a target region of a patient's body. The target region can be a line, a curve, an area, a surface area, or a volume of any arbitrary shape, any complexity, and/or any size. The energy delivery can precisely satisfy therapeutic dose/fluence requirements without requiring a large and expensive energy source. A treatment region can be "painted" or "filled in" by scanning a projected beam of smaller dimension than the treatment region itself, giving precise control over dose and/or fluence, as well as other factors that impact efficacy and/or safety (e.g., dynamic parameters, such as the scan rate, path repetition rate, power modulation rate and the temporal modulation shape (cw, step function, saw-tooth curve, etc.); spectral content of the beam; treatment depth; and the like). In other words, an energy profile in time, space, and wavelength may be precisely delivered to the target region.

The system 20 can combine advantages of pulsed-light with precise large-area/surface area/volume irradiation by scanning a projected energy beam over a precise path. As such, moving the energy beam can be equivalent to pulsing a stationary energy beam in the sense that a particular portion of the patient's body sees the beam for a period of time (which may be brief) and then the beam moves to the next location. Each irradiated location along the path experiences an ON and OFF time. For scans that repeat (over at least a portion of the path), each irradiated location that repeats can experience a sequence of ON and OFF times. Dose and/or fluence can be controlled by adjusting one or more delivery characteristics, including beam intensity, beam dimensions, beam size, beam shape, number of beams, beam modulation, scan rate, path length/shape, path overlap, number of repetitions, or the like. In some instances, the delivery characteristic can be varied along the path.

As shown in FIG. 1, the system 20 includes a beam path determination unit 22 and an energy delivery system 24. The beam path determination unit 22 can include a processor and a non-transitory memory (not shown) so that the beam path determination unit 22 operates as (or has the functionality of) a computer and image processor. The beam path determination unit 22 can be configured to define a path for an energy beam to follow and a duration as it delivers a dose and/or fluence of energy to the target region of the patient's body. At least a portion of the path can be straight and/or curved and open and/or closed and continuous and/or discontinuous. For example, the beam path determination unit 22 can track a path delineated on the patient's skin (e.g., drawn or marked on the patient's skin, indicated by fiducial markers (on the patient's skin, embedded within the patient's tissue, applied to an object near the patient, etc.)/tattoos, etc.). In such instances, the beam path determination unit 22 can perform auto-tracking of the path. As another example, the beam path determination unit 22 can be preprogrammed with at least a portion of the path. As yet another example, the beam path determination unit 22 can process images (generated by radiography, ultrasonography, computed tomography, and the like) to define a path or treatment region based on criteria provided by a clinician.

The beam path determination unit 22 can instruct the energy delivery system 24 of the path and duration to deliver the energy beam. For example, the beam path determination unit 22 can send instructions regarding the path and/or duration to the energy delivery system 24. The energy delivery system 24 can include at least an energy source 26 and a director device 28. The energy source 26 can deliver an energy beam (which may have a focal length that can vary along the path) of a projected shape (a circle, a square, a rectangle, a triangle, a hexagon, or the like) and a projected size. The energy source 26 can include one or more sources that are configured to project at least a portion of the energy beam (in some instances, the portions of the energy beam can differ in wavelength, frequency, spectral content, or the like). For example, the one or more sources can be lasers, laser diodes, light emitting diodes, and/or other types of light sources. The energy beam can be continuous (cw) and/or pulsed and can be subject to spectral adjustment and/or spectral modulation. The director device 28 can be configured to direct the energy beam along the path defined by the beam path determination unit 22.

FIG. 2 shows two examples 10, 11 of beam projection along a path. The beam axis may change orientation in examples 10 and 11 and the path may take any shape in space during the generation of the associated dose profile and/or fluence profile. Note that the dose profile and/or fluence profile will not always be perpendicular to the beam axis.

An example path can be determined by the system of FIG. 1 in which optical energy at a particular wavelength is delivered to the bone marrow that resides within the shoulder blades, spine, and hip bones of a patient. Traditionally, a patch of light is shined, first on one shoulder blade, then on the other, then successively along the spine, and so on. This is time consuming, expensive, and not particularly precise. Using the system 20, instead of "tiling" a patch of light over the entire extended treatment region, a light beam is manipulated and scanned to precisely, specifically, and rapidly address every individual location within the extended target region.

Because the beam is scanned (moving relative to the target region), certain bio-dynamic phenomena that are power- and time-sensitive (such as self-cooling by blood perfusion, photo-bleaching of tissue, chemical and electrical transport within neuronal networks, protein interactions in endosome trafficking, transient absorption and energy transport within and between sub-cellular structures, and the like) may be specifically addressed to improve the efficiency and/or efficacy of the procedure.

The rate at which the beam is scanned can also serve as a precisely selective therapeutic parameter: ensuring interaction with some biological features and remaining "invisible" to others. This is possible because certain biological processes have "time constants" of their own: some processes respond very quickly to energy stimulation and others much more slowly. So that in some cases, a beam can pass so quickly over a treatment region that only certain "fast" or "ultra-fast" biological functions are able to respond, while for other slower processes it is as if the beam was never present at all. In this way, this invention provides a means for providing therapeutic specificity to large and/or complex target regions. Thus the scan rate of this invention can be used to prevent and/or avoid certain slower biological and physical processes (such as heat transfer and thermal damage) while targeting certain faster processes (such as mitochondrial absorption).

For example, because the dwell time of a rapidly scanned beam of this invention can be very short, higher power lasers (which, in certain deep tissue applications, have greater penetration depths than low power energy sources) may be used to treat internal tissues without traumatizing the overlying tissue. If too much energy is put into a region of bodily tissue, the bodily tissue can be heated up and damaged. However, given enough time, the blood flowing through living tissue that has been heated can dissipate heat before additional heat is added to damaging levels. Therefore, powerful energy beams can be delivered into tissue without damaging the tissue if the beam is translated rapidly over the treatment region so as to achieve a dwell time that is therapeutically significant but thermally safe. A rapidly scanned beam can be thought of as being equivalent to a stationary beam that is pulsed ON briefly and then left OFF long enough for blood perfusion to dissipate the heat. Notice however, that unlike a stationary beam, the scanned beam of this invention moves to another location within the treatment region while the previously treated tissue is given sufficient time to normalize after its brief period of irradiation. Furthermore, after each targeted portion of the treatment region has been treated safely and momentarily, the scanning beam can be directed to return again and again to the same locations to effect a total accumulated dose that satisfies the therapeutic endpoint. In this manner, large and complex target regions can be rapidly treated using a powerful beam without adverse effects in any portion of the tissue.

As noted, the translation of the scanning energy beam of this invention results in the intermittent irradiation of a bodily target region. Thus, even though the energy source may be operated in cw, or continuous wave, mode, the target region experiences intermittent or pulsed exposure. This invention therefore can be used to impart bio-dynamic and photo-dynamic specificity to the therapy even when the energy beam is always or mostly ON. In addition to the clinical advantages described above, this can provide economic advantages because CW operation of lasers, laser diodes, light emitting diodes, incandescent light sources, and the like, can be a simple and cost-effective source of energy. However, pulsed energy sources are also entirely compatible with this invention. While the energy beam is translated it may also be pulsed, or time modulated. Time modulation, wherein the energy source or the projected beam is repeatedly turned ON and OFF or modulated so that the beam power follows a square wave, or sine wave, or triangular wave or the like, when used in combination with beam translation, imparts additional degrees of freedom to the therapy which are otherwise unavailable. For example, by scanning and simultaneously pulsing the beam, targeted tissue can be exposed to energy for ultra-short durations which might not be achievable by scanning or pulsing alone.

### Methods

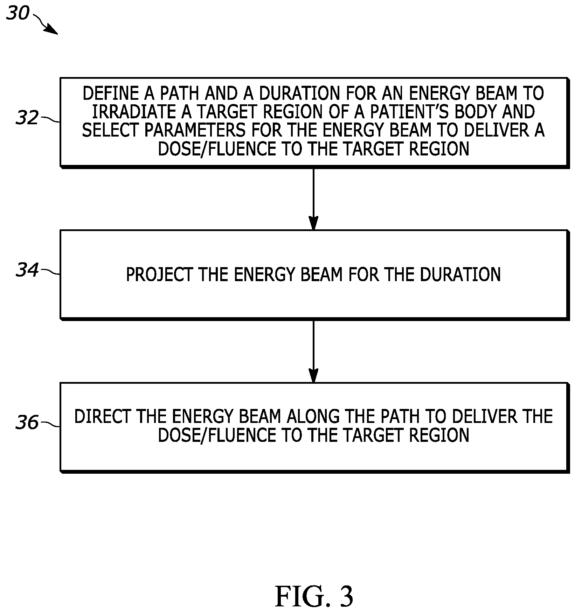

Another aspect of the present disclosure can include a method 30 (FIG. 3) for delivering a dose profile and/or a fluence profile to a target region of a patient's body of a shape and/or a size. The method 30 can be performed using a beam path determination unit 22 and an energy delivery system 24 (e.g., of the system 20 shown in FIG. 1 and described above). Those methods are not a part of the invention.

The method 30 is illustrated as a process flow diagram with flowchart illustrations. For purposes of simplicity, the method 30 shown is and described as being executed serially; however, it is to be understood and appreciated that the present disclosure is not limited by the illustrated order as some steps could occur in different orders and/or concurrently with other steps shown and described herein. Moreover, not all illustrated aspects may be required to implement the method 30.

At 32, a path and a duration (which may vary for different parts of the path) for an energy beam to irradiate a target region of a patient's body can be defined (e.g., by beam path determination unit 22) and parameters can be selected to deliver a dose/fluence (or dose/fluence profile) of energy to a target region of a patient's body (which may be an area, a surface area, and/or a volume). The path and the duration can be defined to satisfy a dose profile and/or a fluence profile (which can be defined during and/or prior to the projection of the beam and/or adjusted during projection of the beam). The defining or adjusting can be based on at least one measurable parameter related to efficacy and/or safety. In some instances, a marked path on the patient's skin (e.g., a fiducial marker, a marking drawn on the patient's skin, etc.) can be tracked to determine the path. In other instances, the beam path can be defined based on a two dimensional (2D) or three dimensional (3D) image of the patient's internal anatomy. In yet other instances, the beam path can be defined or adjusted based on markings or markers or transponders located in, on, or near the patient.

At 34, the energy beam can be projected (e.g., by energy source 26 of energy delivery system 24) for the duration. The dose can be controlled by adjusting a scan rate, a number of repetitions, etc. For example, the energy beam can be projected as a continuous wave (cw) beam, a scanned beam, and/or a pulsed beam. The energy beam can be of a projected shape (a circle, a square, a rectangle, a hexagon, a triangle, or the like) and/or a projected size. The energy beam can be collimated, diverging, converging, focused, diffuse, substantially uniform, and/or Gaussian in profile. At 36, the energy beam can be directed along the path (e.g., by director device 28 of energy delivery system 24) to deliver the dose/fluence (or dose/fluence profile) to the target region of the patient's body. In other words, the energy beam can be scanned (e.g., as a raster scan) along the portions of the path for the duration.

### Theory Scanned Energy Beams

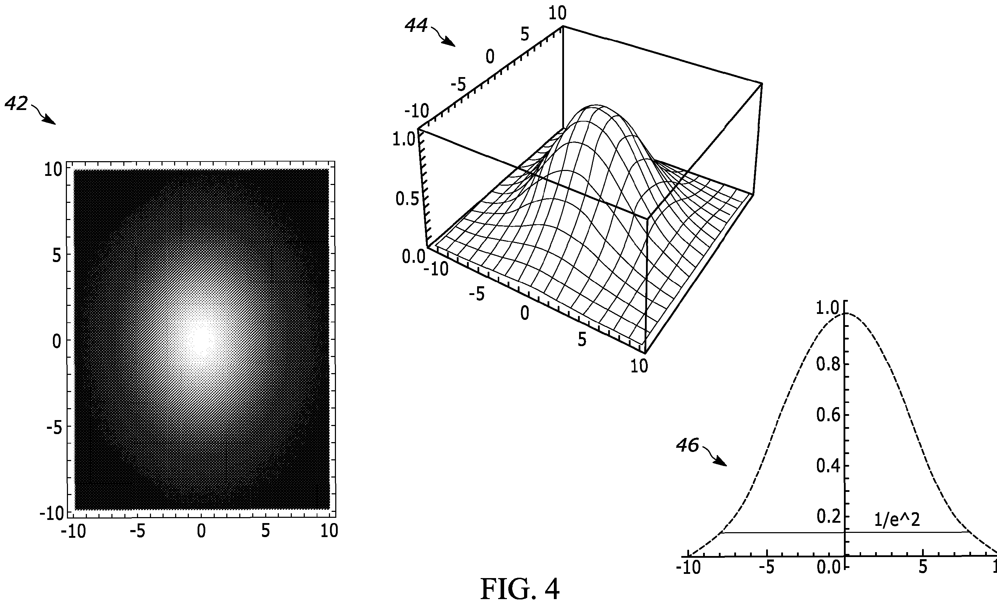

FIG. 4 shows a normalized Gaussian beam profile. The intensity distribution looking directly into the beam is shown at 42. A 3D plot of the intensity distribution is shown at 44. A 2D axial section through the center of the beam is shown at 46, showing intensity and a standard measure of beam size (other standard measures include FWHM, diameter at 90% power, etc.). The terms "beam size" and "spot size" of Gaussian beams refer to any standard measure of the beam size. Non-Gaussian beam profiles (e.g., flat top profiles, bat-wing profiles, triangular profiles, etc.) have similar measures of beam size or spot size. It will be understood that although many of the examples herein use Gaussian beam profiles, the examples can be extended to include non-Gaussian beams.

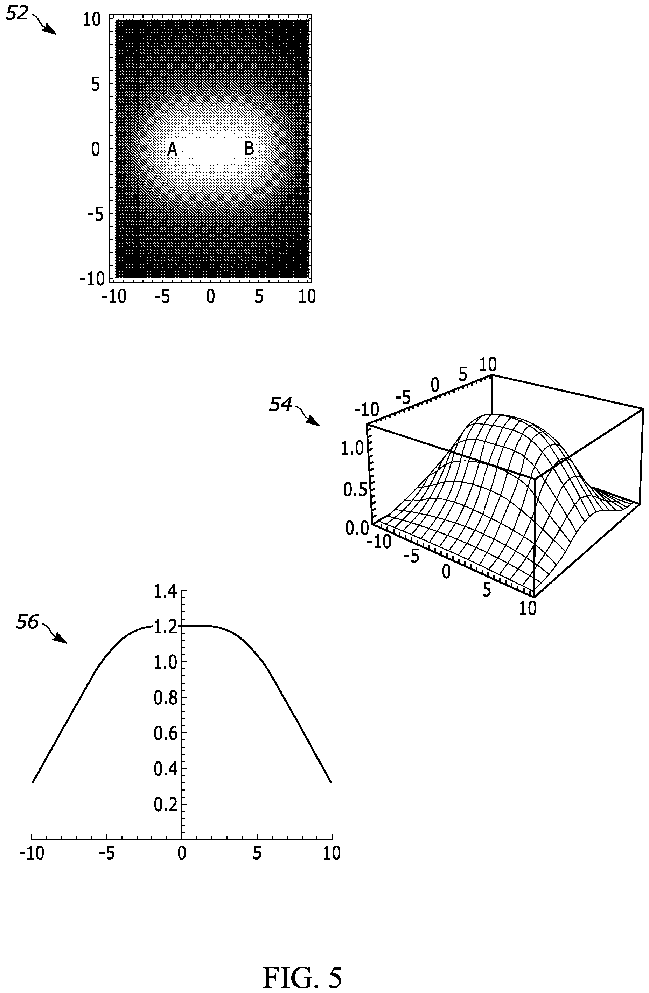

FIG. 5 shows a summation of two neighboring Gaussian beams. Shown at 52 is the normalized intensity distribution of two beams, looking directly into the beams, a first beam centered at point A and a second beam centered at point B. A 3D plot of the normalized intensity distribution of the combination of the two beams is shown at 54. A 2D axial section through the center of the beams is shown at 56. In this simple example, the two beams shine simultaneously and their intensities add together, delivering a total dose to the target region that would have the same general profile as shown in the FIG. 4. A single beam of this invention that first shines at location A and is then translated to location B can deliver the same total dose to the target region as the two beams that shine simultaneously. It is noted that by judicious selection of beam size, beam path, other beam properties (power, spectral content, etc.) and/or scan rates, it is possible to create any shaped dose and/or fluence profile that is desired and which results from the summation within the target tissue of energy over time delivered by a moving beam.

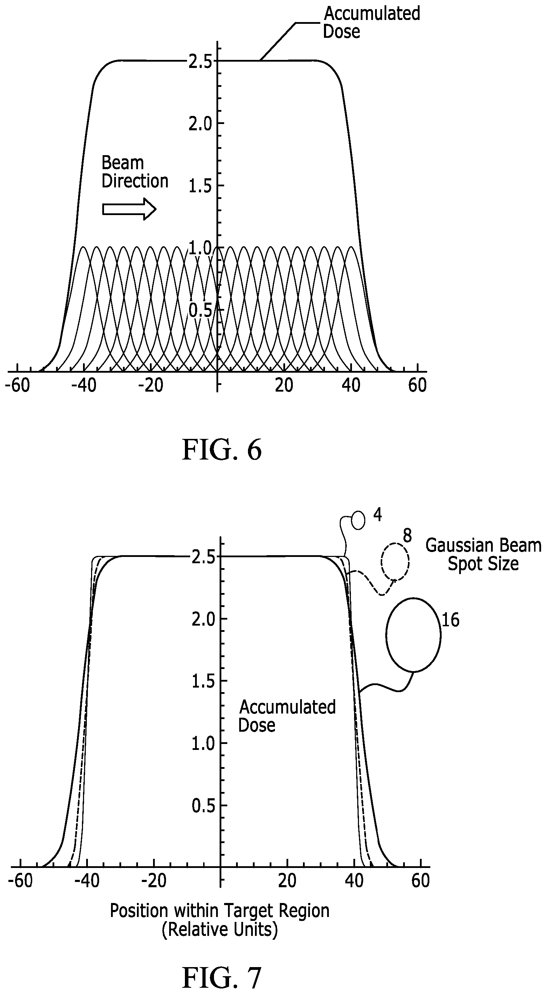

FIG. 6 shows a 2D sectional plot of the translation of a Gaussian beam from left to right and the associated accumulated dose that is delivered to a target region. The translated beam may be scanned repeatedly over the same target region so as to further accumulate the delivered dose. Furthermore, the beam path may be directed anywhere within the 2D or 3D target region and may follow a curved path or any complex path of continuously changing direction.

FIG. 7 shows the relationship between the beam spot size and the sharpness of the accumulated dose profile. Generally, as the beam spot size is decreased, the edges of the dose and/or fluence profile within the treated zone are sharpened. In other words, the beam spot size determines the resolution of the treatment. In any case, the beam spot size must be less than the smallest lateral extent of the treatment region (in the same way that a paint brush must be smaller than the region that is to be painted).

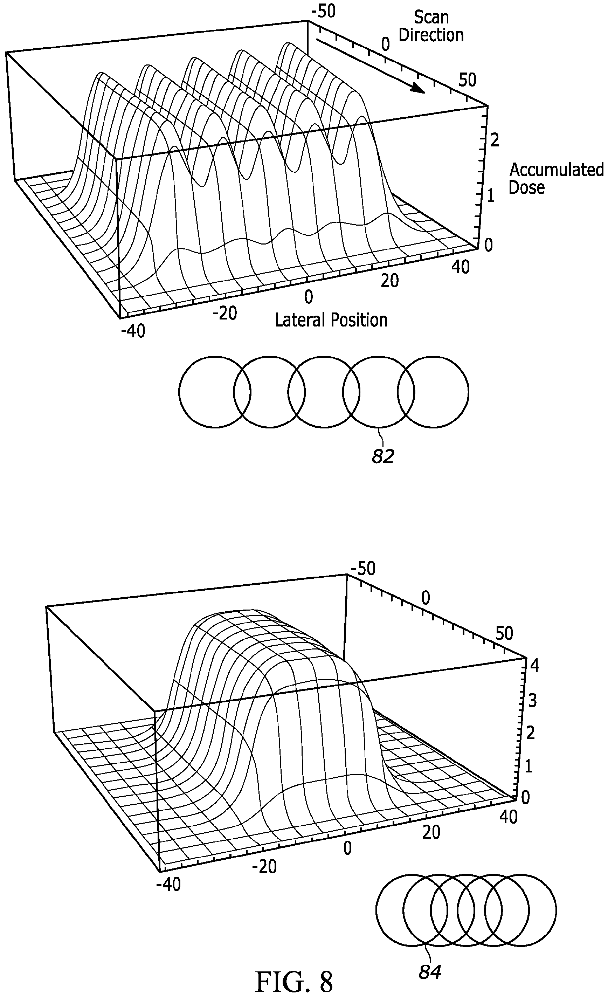

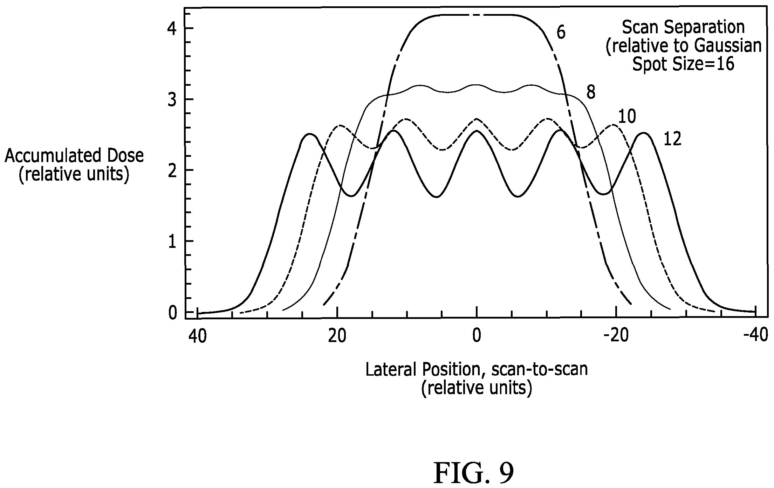

FIG. 8 shows the relationship between the separation of adjacent scan paths and the uniformity of the accumulated dose. At left, five parallel paths of a Gaussian beam of spot size = 16 units 82 are shown; each path is separated from the last by a distance of 12 units. Notice that the accumulated dose exhibits troughs between scans. At right, the same Gaussian beam (spot size = 16 units) is again translated along 5 parallel scan paths 84, but this time the paths are separated by only 6 units. Notice that the accumulated dose is now substantially uniformly distributed within the scanned region, and that the scanned region is smaller than the scanned region in the figure at left. Generally, the uniformity of the accumulated dose profile increases as the ratio of the spot size to the path separation is increased (further illustrated in FIG. 9).

FIG. 9 shows different degrees of uniformity used for different therapeutic applications. Some therapeutic applications will be more forgiving than other therapeutic applications. In fact, some therapeutic applications may require specific non-uniformities. Such specific non-uniformities can be delivered precisely by the system of FIG. 1 through the adjustment of scan rate, scan separation, beam power, beam size and shape, and the number of repeated scans.

### Theory - Energy Delivery to Various Target Regions

Some applications for energy delivery may be directed at skin treatments, which primarily involve the superficial delivery of a therapeutic dosage of energy. In the case of a skin treatment, the treatment region is a surface area. Other applications for energy delivery may require the delivery of a therapeutic dose to organs below the surface of the skin, in which case the treatment region is a volume. It will be understood that when energy is delivered through the skin to underlying tissues, some portion of that energy will be scattered, absorbed, and generally attenuated by the skin and by other intervening tissues. Furthermore, if the beam power is increased to compensate for attenuation, potential skin damage poses an upper limit on beam power.

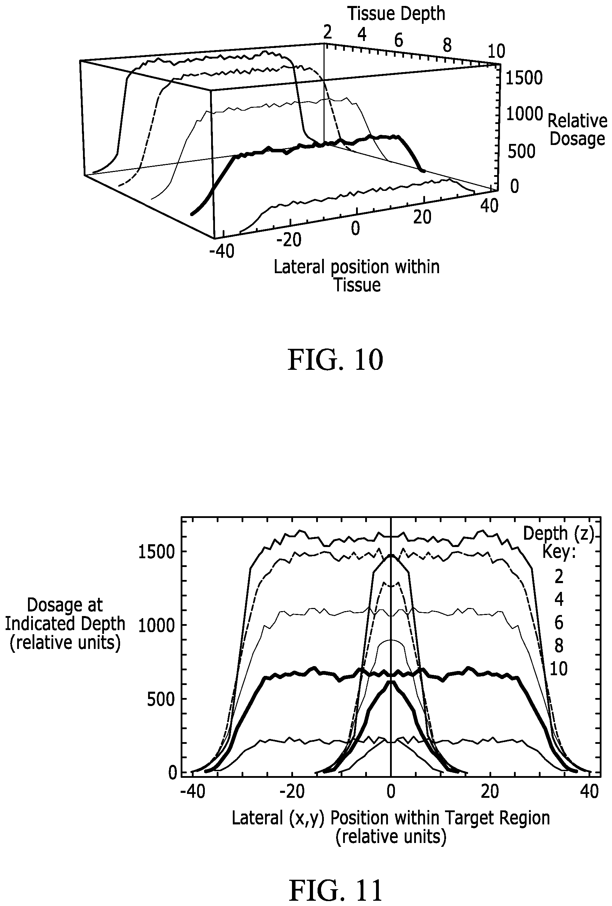

FIGS. 10 and 11 show the attenuation of energy due to scattering as a function of depth. FIG. 10 represents a Monte Carlo simulation of the dosage reduction that results as a flat-top light beam of large spot size penetrates light-scattering tissue. FIG. 11 shows a comparison of the simulation results for two beam sizes: large spot size beam and small spot size beam of the same intensity. Dosage at depth is higher and more uniform for the larger (wider) beam. Thus, optimal dosing of large subcutaneous treatment volumes requires that large areas of the skin's surface be irradiated. However, light beams of sufficient size and power are typically not available for this purpose or are too costly or complex. In this invention, large (wide) irradiation areas are easily achieved by scanning an energy beam. Figures 10 and 11 apply just as well to the scanned beam of this invention as they do to the single stationary beams of the example: in other words, from the standpoint of the attenuation of energy that results as a beam penetrates flesh, a large scanned area of this invention is equivalent to an equally large single beam. Thus, the benefits of depth penetration that are conferred by large and uniform surface irradiation areas are achieved in this invention without the cost and difficulty of delivering a single large and uniform stationary energy beam. Furthermore, in this invention, higher beam powers, that penetrate more deeply, may be delivered without damage to the skin because the scanned beam is always moving.

The beam is translated with a speed (distance/time) and direction across the treatment region. This translation rate has a direct bearing on any treatment parameters that include a time factor, such as irradiance (amount of energy that passes through a unit surface area of the target region per unit time). For example, if a beam operating at a given power level is translated at a Rate A across the treatment surface wherein Rate A is faster than Rate B, then the resulting average irradiance for Rate A will be less than that of Rate B. In addition, because the dwell time of a translated beam on a patient's skin is reduced relative to that of a stationary beam, excessive accumulation of heat during treatment can be prevented. After the translating beam leaves a sub-region of the treatment region, natural heat transport processes, such as blood perfusion, will dissipate any excess heat before the beam is returned to that sub-region for any additional scans. The dissipation of heat by the patient's body is an important safety consideration during photo-treatment. Therefore, beam translation speed provides a means of delivering high powers while simultaneously protecting a patient's skin during treatment. It also remains true that if the beam is translated across the treatment surface at a Power Level A that is half the value of a Power Level B, then the resulting Irradiance for the beam moving at Rate A with Power Level A will be half that of the beam moving at Rate A with Power Level B.

Furthermore, the translating beam may be collimated, diverging, converging, or specifically focused relative to the treatment region. These parameters need not be fixed; they may be changed while the beam is translated as might be required to optimize the intended treatment, for example, for a target region that varies in depth. The translating beam may be continuously ON (cw) and/or modulated (square wave, sinusoidal, etc.) as it is translated along the treatment path. These and other parameters of beam control including spectral content, number of beams, angle of incidence, etc. may all be applied to the beam so as to optimize and adjust the quality of treatment.

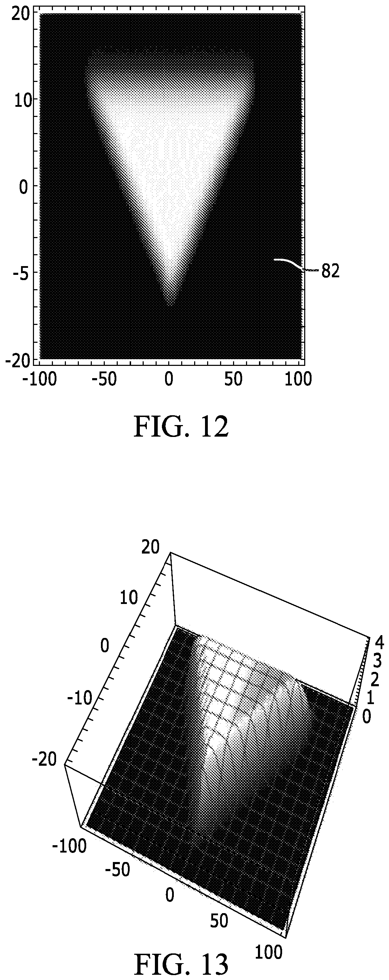

This disclosure enables delivery of precise dose, fluence, and irradiance levels to regions that are clinically large and/or complex. FIGS. 12 and 13 show a triangular treatment zone achieved by translation of a Gaussian beam. FIG. 12 shows the normalized axial (or top) view of the accumulated dose. FIG. 13 shows a 3D plot of the normalized dose as a function of the x and y positions within the treatment zone. The well-defined edges of the treatment zone were achieved with a Gaussian beam spot size of 8 units (relative to the scale shown along the x and y axes).

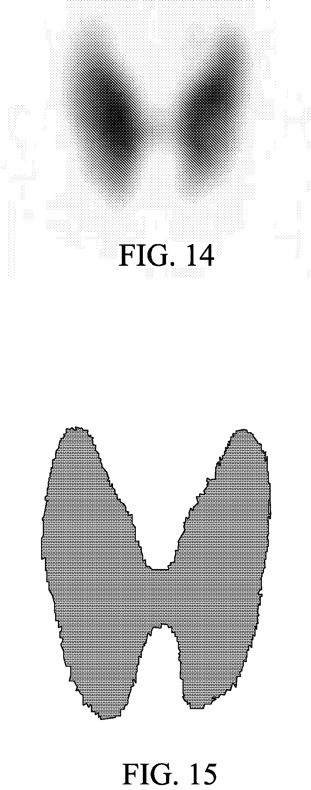

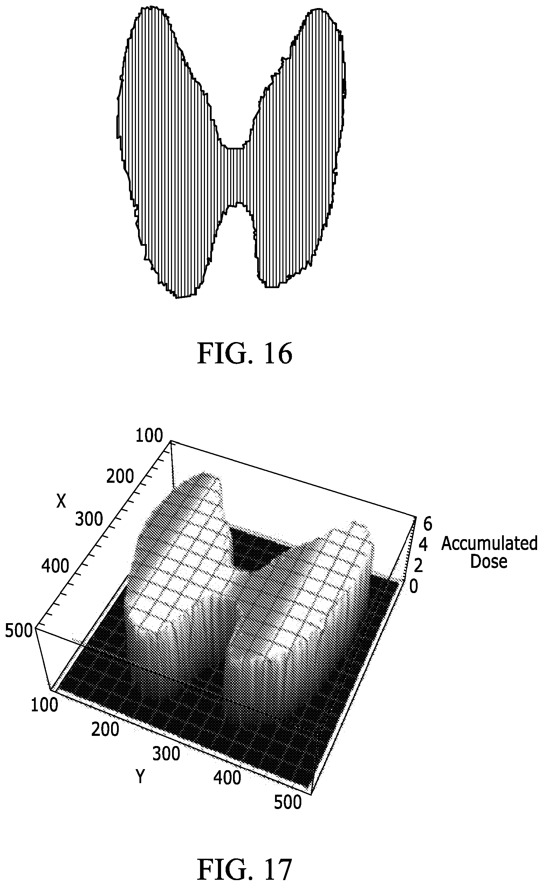

Determination of the beam translation path is made in accordance with the shape and size of the region to be treated, the desired degree of dose uniformity to be achieved, and the desired resolution or sharpness of the delivered dose region. As an example, a clinician may wish to apply photo-treatment to the thyroid of a patient. A preliminary radionuclide scan of the thyroid is acquired using standard lab techniques (FIG. 14). The clinician, in conjunction with an image processing unit (e.g., of the beam-path determination unit 12) defines the region to be treated (FIG. 15). A preferred path is generated (e.g., by the beam-path determination unit 12), as represented by the white lines in FIG. 16. Both the spacing of the parallel paths and the beam spot size are selected to achieve the degree of dose uniformity and the sharpness of the delivered dose region that are required to treat the thyroid. The highly uniform dose distribution shown in the 3D plot of FIG. 17 results from a Gaussian beam spot size of 8 units and path separation of 4 units (relative to the scale shown along the x and y axes). Further optimization of this profile may be achieved, for example, by adjusting the beam power as a function of scan position so as to deliver a higher or lower dose to selected sub-regions, as needed.

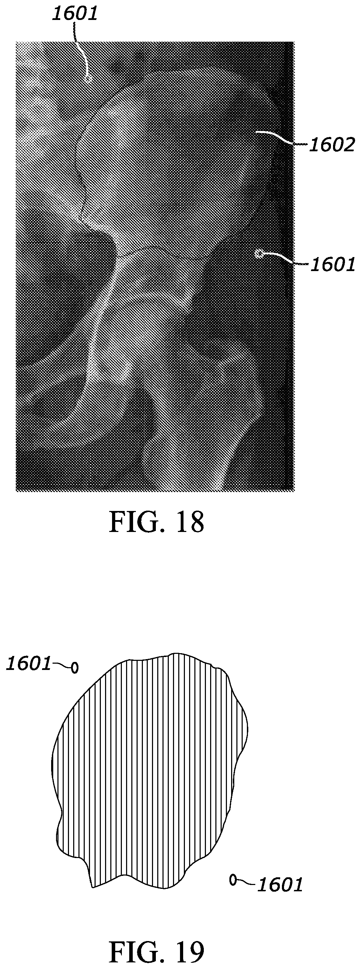

FIGS. 18 and 19 show fiducial markers being used to effect precise registration of the beam path with the intended surface treatment zone or the intended deep tissue treatment zone. The region to be treated may be identified prior to treatment or during treatment. For example, x-ray imaging might be used to provide 2D or 3D information about target structures during treatment, and this information can be used by the clinician in conjunction with the beam path determination unit 22 to generate an adaptive path that adjusts, for example, to patient motion. Furthermore, fiducial markers, skin surface markings, tattoos, or the like may be used (e.g., by the beam path determination unit 22) to effect precise registration of the beam path with the intended surface treatment zone or the intended deep tissue treatment zone. FIG. 18 shows an x-ray image of a patient's left Ilium with two fiducial markers 1601 and clinician's outline of intended treatment area 1602. An example representation of the treatment zone, beam path, and fiducial marker information is shown in FIG. 19.

It should be noted that any well-known or developing method(s) can be used for the scanning of energy beams (e.g., galvanometric, acousto-optic, flat-bed translation, dynamic lenses, phasing of multi-element arrays, etc.). Thus, very large treatment regions, spanning any portion of the human body and focused to any depth within the body, may be addressed.

The invention is defined in the claims.

## Claims

1. A system (20) that delivers a precisely contoured dose profile and/or fluence profile to a target region of a patient's body of a shape or a size, comprising:
a beam-path determination unit (22) configured to define a scan pattern, comprising one or more paths, of an electromagnetic beam to deliver the precisely contoured dose profile and/or fluence profile to the target region, wherein the dose profile or fluence profile is precisely contoured by configuring parameters of the electromagnetic beam, wherein the parameters comprise a scan rate and a beam power delivered at the scan rate for intermittent irradiation of the target region, wherein the scan rate is coupled with a beam power modulation rate to effect irradiation of the target region at the beam power; and
an energy delivery system (24) comprising an energy source (26) and director device (28), coupled to the beam-path determination unit (22), wherein the energy source (26) is configured to project the electromagnetic beam with the parameters and the director device (26) is configured to direct the electromagnetic beam along the scan pattern to accumulate energy in the target region, resulting in the intermittent irradiation of the target region.

2. The system (20) of claim 1, wherein the scan pattern is determined at least in part by external guides comprising at least one of: markings or markers placed on, in, or around the target region; transponders; images acquired during a scanning process, and
wherein the path determination unit (22) includes one or more guide sensors and/or guide image processors.

3. The system (20) of claim 1, wherein the electromagnetic beam has a Gaussian intensity profile.

4. The system (20) of claim 1, wherein at least a portion of the parameters are varied by the energy delivery system along the one or more paths.

5. The system (20) of claim 1, wherein the target region of the patient's body is an area, a surface area, and/or a volume.

6. The system (20) of claim 1, wherein the dose profile and/or the fluence profile is defined as the electromagnetic beam is directed along the one or more paths based on at least one measurable efficacy parameter and/or safety parameter.

7. The system (20) of claim 1, wherein the electromagnetic beam comprises a focal length, a projected shape, and/or projected size.

8. The system (20) of claim 1, wherein the electromagnetic beam is projected along the path with a number of repetitions of at least a portion of the path.

9. The system (20) of claim 8, wherein the energy source facilitates spectral adjustment and/or spectral modulation of the energy beam.

10. The system (20) of claim 1, wherein the beam-path determination unit (22) defines the one or more paths based on a marking and/or marker on a patient's skin, embedded within the patient's tissue, or applied to an object near the patient.

11. The system (20) of claim 10, wherein the marking and/or marker comprises at least one fiducial marker and/or a drawing.

12. The system (20) of claim 1, wherein the beam-path determination unit (22) defines the one or more paths based on a 2D image or a 3D image of a patient's internal anatomy.

13. The system (20) of claim 1, wherein the precisely contoured dose profile and/or the fluence profile is defined prior to projection of the electromagnetic beam.

14. The system (20) of claim 1, wherein the parameters further comprise at least one of beam size, separation distance and/or overlap of sequential paths, and a number of path repetitions.

## Patentansprüche

1. System (20), das ein präzise konturiertes Dosisprofil und/oder Fluenzprofil an eine Zielregion eines Körpers eines Patienten mit einer Form oder einer Größe abgibt, umfassend:
eine Strahlwegbestimmungseinheit (22), die dazu konfiguriert ist, ein Abtastmuster, umfassend einen oder mehrere Wege, eines elektromagnetischen Strahls zu definieren, um das präzise konturierte Dosisprofil und/oder Fluenzprofil an die Zielregion abzugeben, wobei das Dosisprofil oder das Fluenzprofil durch Konfigurieren von Parametern des elektromagnetischen Strahls präzise konturiert wird, wobei die Parameter eine Abtastrate und eine Strahlleistung, die mit der Abtastrate abgegeben wird, zur intermittierenden Bestrahlung der Zielregion umfassen, wobei die Abtastrate mit einer Strahlleistungsmodulationsrate gekoppelt ist, um eine Bestrahlung der Zielregion mit der Strahlleistung zu bewirken; und
ein Energieabgabesystem (24), umfassend eine Energiequelle (26) und eine Richtervorrichtung (28), die an die Strahlwegbestimmungseinheit (22) gekoppelt sind, wobei die Energiequelle (26) dazu konfiguriert ist, den elektromagnetischen Strahl mit den Parametern zu projizieren, und die Richtervorrichtung (26) dazu konfiguriert ist, den elektromagnetischen Strahl entlang dem Abtastmuster zu richten, um Energie in der Zielregion zu akkumulieren, was zu der intermittierenden Bestrahlung der Zielregion führt.

2. System (20) nach Anspruch 1, wobei das Abtastmuster zumindest zum Teil durch externe Führungen bestimmt wird, umfassend mindestens eine bzw. einen von: Markierungen oder Markern, die auf der, in der oder um die Zielregion platziert sind; Transpondern; während eines Abtastvorgangs aufgenommenen Bildern, und
wobei die Wegbestimmungseinheit (22) einen oder mehrere Führungssensoren und/oder Führungsbildprozessoren beinhaltet.

3. System (20) nach Anspruch 1, wobei der elektromagnetische Strahl ein Gaußsches Intensitätsprofil aufweist.

4. System (20) nach Anspruch 1, wobei mindestens ein Teil der Parameter von dem Energieabgabesystem entlang dem einen oder den mehreren Wegen variiert wird.

5. System (20) nach Anspruch 1, wobei die Zielregion des Körpers des Patienten ein Bereich, ein Oberflächenbereich und/oder ein Volumen ist.

6. System (20) nach Anspruch 1, wobei das Dosisprofil und/oder das Fluenzprofil auf der Basis mindestens eines messbaren Wirksamkeitsparameters und/oder Sicherheitsparameters definiert wird, während der elektromagnetische Strahl entlang dem einen oder den mehreren Wegen gerichtet wird.

7. System (20) nach Anspruch 1, wobei der elektromagnetische Strahl eine Brennweite, eine projizierte Form und/oder eine projizierte Größe umfasst.

8. System (20) nach Anspruch 1, wobei der elektromagnetische Strahl entlang dem Weg mit einer Anzahl von Wiederholungen mindestens eines Teils des Wegs projiziert wird.

9. System (20) nach Anspruch 8, wobei die Energiequelle eine spektrale Anpassung und/oder eine spektrale Modulation des Energiestrahls erleichtert.

10. System (20) nach Anspruch 1, wobei die Strahlwegbestimmungseinheit (22) den einen oder die mehreren Wege auf der Basis einer Markierung und/oder eines Markers auf der Haut eines Patienten, innerhalb des Gewebes des Patienten eingebettet oder auf ein Objekt nahe dem Patienten aufgebracht, definiert.

11. System (20) nach Anspruch 10, wobei die Markierung und/oder der Marker mindestens einen Bezugsmarker und/oder eine Zeichnung umfasst.

12. System (20) nach Anspruch 1, wobei die Strahlwegbestimmungseinheit (22) den einen oder die mehreren Wege auf der Basis eines 2D-Bilds oder eines 3D-Bilds einer inneren Anatomie eines Patienten definiert.

13. System (20) nach Anspruch 1, wobei das präzise konturierte Dosisprofil und/oder das Fluenzprofil vor der Projektion des elektromagnetischen Strahls definiert wird bzw. werden.

14. System (20) nach Anspruch 1, wobei die Parameter weiterhin mindestens eine bzw. einen von einer Strahlgröße, einem Trennungsabstand und/oder einer Überlappung von sequenziellen Wegen und einer Anzahl von Wegwiederholungen umfassen.

## Revendications

1. Système (20) apportant un profil de dose et/ou un profil de fluence aux contours définis avec précision à une région cible du corps d'un patient d'une certaine forme ou d'une certaine taille, comprenant :
une unité de détermination de trajet de faisceau (22) configurée pour définir un diagramme de balayage, comprenant un ou plusieurs trajets d'un faisceau électromagnétique pour apporter le profil de dose et/ou le profil de fluence aux contours définis avec précision à la région cible, le profil de dose ou le profil de fluence ayant des contours définis avec précision par configuration de paramètres du faisceau électromagnétique, les paramètres comprenant une vitesse de balayage et une puissance de faisceau apportée à la vitesse de balayage pour une irradiation intermittente de la région cible, la vitesse de balayage étant couplée à une vitesse de modulation de puissance de faisceau pour effectuer une irradiation de la région cible à la puissance du faisceau ; et
un système d'apport d'énergie (24) comprenant une source d'énergie (26) et un dispositif directeur (28), couplés à l'unité de détermination de trajet de faisceau (22), la source d'énergie (26) étant configurée pour projeter le faisceau électromagnétique conforme aux paramètres et le dispositif directeur (26) étant configuré pour diriger le faisceau électromagnétique le long du diagramme de balayage afin d'accumuler de l'énergie dans la région cible, en entraînant une irradiation intermittente de la région cible.

2. Système (20) selon la revendication 1, dans lequel le diagramme de balayage est déterminé au moins en partie par des guides externes comprenant au moins l'un de : marquages ou repères placés sur, dans, ou autour de la région cible ; transpondeurs; images acquises au cours d'un processus de balayage, et
dans lequel l'unité de détermination de trajet (22) comprend un ou plusieurs capteurs de guidage et/ou processeurs d'images de guidage.

3. Système (20) selon la revendication 1, dans lequel le faisceau électromagnétique a un profil d'intensité gaussien.

4. Système (20) selon la revendication 1, dans lequel le système d'apport d'énergie fait varier au moins une partie des paramètres le long des un ou plusieurs trajets.

5. Système (20) selon la revendication 1, dans lequel la région cible du corps du patient est une zone, une aire de surface et/ou un volume.

6. Système (20) selon la revendication 1, dans lequel le profil de dose et/ou le profil de fluence sont définis comme le faisceau électromagnétique qui est dirigé le long des un ou plusieurs trajets sur la base d'au moins un paramètre d'efficacité et/ou paramètre de sécurité mesurable.

7. Système (20) selon la revendication 1, dans lequel le faisceau électromagnétique comprend une distance focale, une forme projetée, et/ou une taille projetée.

8. Système (20) selon la revendication 1, dans lequel le faisceau électromagnétique est projeté le long du trajet avec un certain nombre de répétitions d'au moins une partie du trajet.

9. Système (20) selon la revendication 8, dans lequel la source d'énergie facilite l'ajustement spectral et/ou la modulation spectrale du faisceau d'énergie.

10. Système (20) selon la revendication 1, dans lequel l'unité de détermination de trajet de faisceau (22) définit les un ou plusieurs trajets sur la base d'un marquage et/ou d'un repère sur la peau d'un patient, incorporé dans le tissu du patient, ou appliqué sur un objet à proximité du patient.

11. Système (20) selon la revendication 10, dans lequel le marquage et/ou le repère comprennent au moins un point repère et/ou un dessin.

12. Système (20) selon la revendication 1, dans lequel l'unité de détermination de trajet de faisceau (22) définit les un ou plusieurs trajets sur la base d'une image 2D ou d'une image 3D de l'anatomie interne d'un patient.

13. Système (20) selon la revendication 1, dans lequel le profil de dose et/ou le profil de fluence aux contours définis avec précision sont définis avant la projection du faisceau électromagnétique.

14. Système (20) selon la revendication 1, dans lequel les paramètres comprennent en outre au moins l'un d'une taille de faisceau, d'une distance de séparation et/ou d'un chevauchement de trajets séquentiels, et d'un nombre de répétitions de trajet.
